(19) Europäisches Patentamt
European Patent Office
Office européen des brevets

(11) Veröffentlichungsnummer: **0 363 308 B1**

(12) **EUROPÄISCHE PATENTSCHRIFT**

(45) Veröffentlichungstag der Patentschrift: **01.06.94**

(51) Int. Cl.[5]: **C07C 43/29**, C07C 41/16, C07C 43/295, A01N 31/16

(21) Anmeldenummer: **89810148.0**

(22) Anmeldetag: **27.02.89**

(54) **Neue Aether.**

(30) Priorität: **15.09.88 CH 3444/88**

(43) Veröffentlichungstag der Anmeldung:
**11.04.90 Patentblatt 90/15**

(45) Bekanntmachung des Hinweises auf die Patenterteilung:
**01.06.94 Patentblatt 94/22**

(84) Benannte Vertragsstaaten:
**AT BE CH DE ES FR GB GR IT LI NL**

(56) Entgegenhaltungen:
**EP-A- 0 138 359**
**DE-A- 2 304 962**
**DE-A- 2 527 352**
**GB-A- 2 205 096**

**PATENT ABSTRACTS OF JAPAN, Band 11, Nr. 43 (C-402)(2490), 7. Februar 1987; & JP-A-61 207 352**

**PESTICIDE SCIENCE, Band 14, Nr. 5, Oktober 1983, S. 461-469, Oxford, GB; P. MASSARDO et al: "Synthesis and Juvenile Hormone Activities of Some New Ether Derivatives of Hydroquinone"**

**Journal of Labelled Compounds and Radiopharmaceuticals, XXV, 9, 1007-1015 (1988)**

(73) Patentinhaber: **CIBA-GEIGY AG**
**Klybeckstrasse 141**
**CH-4002 Basel(CH)**

(72) Erfinder: **Karrer, Friedrich, Dr.**
**Rebbergstrasse 5**
**CH-4800 Zofingen(CH)**
Erfinder: **Rindlisbacher, Alfred**
**Lachmattstrasse 69/3**
**CH-4132 Muttenz(CH)**

**EP 0 363 308 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft 1-[4-(Halophenoxy)-phenoxy]-4-pentine, Verfahren und Zwischenprodukte zu ihrer Herstellung und ihre Verwendung in der Schädlingsbekämpfung.

Die erfindungsgemässen Verbindungen entsprechen der Formel I

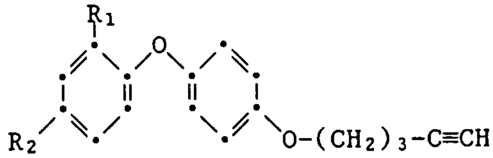

(I)

worin $R_1$ und $R_2$ voneinander unabhängig Chlor oder Fluor bedeuten und entweder $R_1$ oder $R_2$ auch Wasserstoff bedeutet.

Von den Verbindungen der Formel I sind für die Schädlingsbekämpfung sowohl die Mono- und Difluor als auch die Mono- und Dichlorverbindungen bevorzugt.

Als Einzelverbindungen sind hervorzuheben:
das 1-[4-(4-Fluorphenoxy)-phenoxy]-4-pentin,
1-[4-(2-Fluorphenoxy)-phenoxy]-4-pentin und das
1-[4-(2,4-Difluorphenoxy)-phenoxy]-4-pentin.

Substituierte Phenoxy-4-pentine sind aus der DE-OS 2,304,962 als Akarizide bekannt. Von den durch die Definition miterfassten 1-[4-(Monohalophenoxy)-phenoxy]-4-pentinen sind in dieser Publikation jedoch keine Einzelverbindungen beschrieben worden. Schliesslich sind der DE-OS 2,305,698 substituierte Phenoxy-2-pentine, und in der DE-OS 2,547,146 substituierte (4-Phenoxy-phenoxyalkyl)-(alkinyl)-äther und -thioäther mit akarizider Wirkung beschrieben.

Diese Verbindungen befriedigten nicht immer im Hinblick auf ihre akarizide und vor allem ovizide Wirkung.

Ziel der vorliegenden Erfindung ist, weitere Akarizide mit verbesserter Wirkung bereitzustellen.

Es wurde überraschend gefunden, dass die neuen Verbindungen der Formel I eine verbesserte akarizide, vor allem ovizide Wirkung gegenüber dem Stand der Technik aufweisen.

Ebenfalls überraschend ist die ovizide Aktivität der Verbindungen der Formel I gegen Blattläuse, namentlich gegen Dysaphis plantaginea (Mehlige Apfellaus), Aphis pomi und Dysaphis brancoi (Apfelfaltenlaus).

Die Verbindungen der Formel I können durch Umsetzung eines 4-(Halophenoxy)-phenolats der Formel II, worin $R_1$ und $R_2$ die unter Formel I angegebenen Bedeutung haben und M für ein Alkali oder Erdalkalimetall steht mit einem 1-Halo-4-pentin der Formel III, worin $R_3$ Chlor, Brom oder Jod bedeutet.

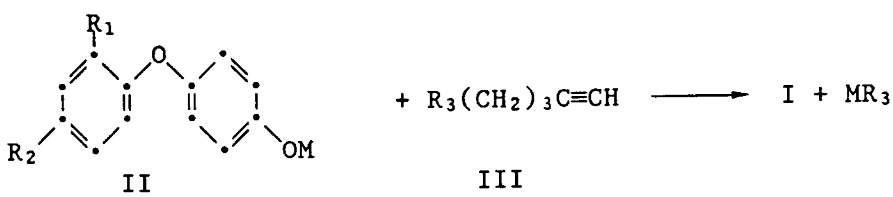

II III

in einem inerten Lösungsmittel, zwischen -10 und +140 °C hergestellt werden.

Das Verfahren wird bevorzugt, wenn es in Gegenwart eines Alkalijodids in einen polaren aprotischen Lösungsmittel wie z.B. Dimethylsulfoxid, Sulfolan oder einem Dialkylamid wie z.B. Dimethylformamid als Lösungsmittel bei 0-80 °C durchgeführt wird und wenn $R_3$ Chlor bedeutet.

Das Verfahren zur Herstellung der Verbindungen der Formel I ist auch Gegenstand der vorliegenden Erfindung.

Das Zwischenprodukt 4-(2,4-Difluorphenoxy)-phenol kann durch Umsetzung des 4-(2,4-Difluorphenoxy)-anisols mit einer wässrigen Halogenwasserstoffsäure, bevorzugt 30-50 %-iger Bromwasserstoffsäure oder Jodwasserstoffsäure bei 80-130 °, vorzugsweise bei 100-110 °, vorzugsweise in Eisessig als Lösungsmittel hergestellt werden.

Das neue 4-(2,4-Difluorphenoxy)-phenol und seine Herstellung sind ebenfalls Gegenstand der vorliegenden Anmeldung.

Das Phenolat der Formel II kann aus dem 4-(2,4-Difluorphenoxy)-phenol und einer geeigneten Base, wie beispielsweise Alkali- oder Erdalkalihydroxide, Alkali- oder Erdalkalialkoholate, Alkali- oder Erdalkalihydride und schliesslich Natrium- oder Kaliumcarbonat nach bekannten Methoden hergestellt und gegebenenfalls isoliert werden.

Die Ausgangsprodukte der Formeln II und III sind bekannt und können, falls sie neu sind, nach bekannten Methoden hergestellt werden.

Die erfindungsgemässen Verbindungen sind bei günstiger Warmblüter- und Pflanzenverträglichkeit wertvolle Wirkstoffe in der Schädlingsbekämpfung. So eignen sich die Verbindungen der Formel I z.B. zur Bekämpfung von Schädlingen an Tieren und Pflanzen. Solche Schädlinge gehören hauptsächlich dem Stamm der Arthropoden an, wie insbesondere Insekten der Ordnungen Lepidoptera, Coleoptera, Homoptera, Heteroptera, Diptera, Thysanoptera, Orthoptera, Anoplura, Siphonaptera, Mallophaga, Thysanura, Isoptera, Psocoptera oder Hymenoptera und Arachniden der Ordnung Acarina, wie z.B. Milben und Zecken, insbesondere die Obstbaumspinnmilbe (Panonychus ulmi), sowie Blattläuse sind zu nennen. Dabei kann jedes Entwicklungsstadium der Schädlinge bekämpft werden, d.h. sowohl die Adulten, Puppen und Nymphen, als auch insbesondere die Larven und vorzugsweise die Eier. So können vor allem Larven und Eier von phytopathogenen Schadinsekten und Milben in Zier- und Nutzpflanzungen, wie z.B. in Gemüse- und Baumwollpflanzungen und insbesondere in Obstpflanzungen wirkungsvoll bekämpft werden. Die Bekämpfung präimaginaler Stadien von pflanzenschädigenden Insekten ist dabei hervorzuheben. Werden Verbindungen der Formel I von Imagines aufgenommen, so kann sich ihre Wirkung in unmittelbarer Abtötung der Schädlinge zeigen oder aber in verminderter Eiablage und/oder verminderter Schlupfrate.

Bei der Bekämpfung von tierparasitären Schädlingen, insbesondere an Haus- und Nutztieren, kommen vor allem Ektoparasiten, wie z.B. Milben und Zecken und Dipteren, wie z.B. Lucilia sericata in Betracht.

Die gute pestizide Wirkung der erfindungsgemässen Verbindungen der Formel I entspricht einer Abtötungsrate (Mortalität) von mindestens 50-60 % der erwähnten Schädlinge.

Die erfindungsgemässen Verbindungen sind speziell für die Bekämpfung von Obstbaumspinnmilben der Gattung Panonychus insbesondere der Art Panonychus ulmi, bedeutend.

Zur Bekämpfung dieses Schädlings im Präimaginalstadium, dessen Eier in Obstbäumen überwintern und dessen erste Generation im Obstbau besonders viel Schaden verursacht, erfolgt in der landwirtschaftlichen Praxis normalerweise durch Versprühen von oviziden Mitteln gemäss dem Stand der Technik in einer Konzentration von 50 g/100 l auf die Kulturpflanze noch vor der Blüte, worauf nach 2-3 Wochen die Bekämpfung der Schädlinge in den mobilen Entwicklungsphasen mit anderen geeigneten Mitteln zu folgen hat.

Demgegenüber sichern die erfindungsgemässen Wirksubstanzen nach Versprühen in nur 30 g/100 l Konzentration vor der Blüte einen umfassenden Schutz gegen die Obstbaumspinnmilbe von über 6 Wochen, was die praktisch vollständige Abtötung der Schädlinge bedeutet.

Die Wirkung der Verbindungen der Formel I ist ausserordentlich breit; ihre ovizide Wirkung ist sowohl gegen die Obstbaumspinnmilbe als auch gegen die Mehlige Apfellaus und gegen die Apfelfaltenlaus sehr stark.

Die Wirkung der erfindungsgemässen Verbindungen bzw. der sie enthaltenden Mittel lässt sich durch Zusatz von anderen Insektiziden und/oder Akariziden wesentlich verbreitern und an gegebene Umstände anpassen. Als Zusätze kommen z.B. Vertreter der folgenden Wirkstoffklassen in Betracht: Organische Phosphorverbindungen, Nitrophenole und Derivate, Formamidine, Harnstoffe, Carbamate, Pyrethroide, chlorierte Kohlenwasserstoffe und Bacillus thuringiensis-Präparate.

Die Verbindungen der Formel I werden in unveränderter Form oder vorzugsweise zusammen mit den in der Formulierungstechnik üblichen Hilfsmitteln eingesetzt und können daher z.B. zu Emulsionskonzentraten, direkt versprüh- oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet werden. Die Anwendungsverfahren, wie Versprühen, Vernebeln, Verstäuben, Verstreuen oder Giessen, werden ebenso wie die Mittel den angestrebten Zielen und den gegebenen Verhältnissen entsprechend gewählt.

Die Formulierung, d.h. die den Wirkstoff der Formel I, bzw. Kombinationen dieser Wirkstoffe mit anderen Insektiziden oder Akariziden, und gegebenenfalls einen festen oder flüssigen Zusatzstoff enthaltenden Mittel, Zubereitungen oder Zusammensetzungen, werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester, wie Dibutyl- oder Dioctylphthalat, aliphatische Kohlenwasserstoffe, wie Cyclohexan, Paraffine, Alkohole und Glykole sowie deren Aether und Ester, wie Aethanol, Aethylenglykol, Aethylenglykolmonomethyl- oder -äthyläther, Ketone, wie Cyclohexanon, stark polare Lösungsmittel, wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl, oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäuren oder hochdisperse saugfähige Polymerisate zugesetzt werden. Als gekörnte, adsorptive Granulatträger kommen poröse Typen, wie z.B. Bimsstein, Ziegelbruch, Sepiolit oder Bentonit, als nicht sorptive Trägermaterialien z.B. Calcit oder Sand in Frage. Darüberhinaus kann eine Vielzahl von granulierten Materialien anorganischer oder organischer Natur, wie insbesondere Dolomit oder zerkleinerte Pflanzenrückstände, verwendet werden.

Als oberflächenaktive Verbindungen kommen je nach Art des zu formulierenden Wirkstoffes der Formel I oder der Kombinationen dieser Wirkstoffe mit andern Insektiziden oder Akariziden nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen eignen sich die Alkali-, Erdalkali- oder gegebenenfalls substituierten Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Tallöl gewonnen werden können. Ferner sind als Tenside auch die Fettsäure-methyl-taurin-salze sowie modifizierte und nicht modifizierte Phospholipide zu erwähnen.

Häufiger werden jedoch sogenannte synthetische Tenside verwendet, insbesondere Fettsulfonate, Fettsulfate, sulfonierte Benzimidazol-derivate oder Alkylarylsulfonate.

Die Fettsulfonate oder -sulfate liegen in der Regel als Alkali-, Erdalkali- oder gegebenenfalls substituierte Ammoniumsalze vor und weisen im allgemeinen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2 Sulfonsäuregruppen und einen Fettsäurerest mit etwa 8-22 C-Atomen. Alkylarylsulfonate sind z.B. die Na-, Ca- oder Triäthanolaminsalze der Dodecylbenzolsulfonsäure, der Dibutylnaphthalinsulfonsäureoder eines Naphthalinsulfonsäure-Formaldehydkondensationsproduktes. Ferner kommen auch entsprechende Phosphate, wie z.B. Salze des Phosphorsäureesters eines p-Nonylphenol-(4-14)-Aethylenoxid-Adduktes, in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können. Weiterhin geeignete nichtionische Tenside sind die wasserlöslichen 20 bis 250 Aethylenglykoläthergruppen und 10 bis 100 Propylenglykoläthergruppen enthaltenden Polyäthylenoxid-Addukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykol-Einheiten.

Als Beispiele nichtionischer Tenside seien Nonylphenolpolyäthoxyäthanole, Ricinusölpolyglykoläther, Ricinusölthioxilat, Polypropylen-Polyäthylenoxid-Addukte, Tributylphenoxypolyäthoxyäthanol, Polyäthylenglykol und Octylphenoxypolyäthoxyäthanol erwähnt. Ferner kommen auch Fettsäureester von Polyoxyäthylensorbitan, wie das Polyoxyäthylensorbitan-trioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyl-di-(2-chloräthyl)-äthylammoniumbromid.

Die in der Formulierungstechnik gebräuchlichen Tenside sind u.a. in folgenden Publikationen beschrieben:

"Mc Cutcheon's Detergents and Emulsifiers Annual" MC Publishing Corp., Ridgewood, New Jersey, 1979;

Dr. Helmut Stache "Tensid Taschenbuch", Carl Hanser Verlag München/Wien 1981.

Die pestiziden Zubereitungen enthalten in der Regel 0,1 bis 99 %, insbesondere 0,1 bis 95 %, Wirkstoff der Formel I oder Kombinationen dieser Wirkstoffe mit andern Insektiziden oder Akariziden, 1 bis 99,9 % eines festen oder flüssigen Zusatzstoffes und 0 bis 25 %, insbesondere 0,1 bis 20 %, eines Tensides. Während als Handelsware eher konzentrierte Mittel bevorzugt werden, verwendet der Endverbraucher in der Regel verdünnte Zubereitungen, die wesentlich geringere Wirkstoffkonzentrationen aufweisen.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Beispiele: Herstellung der Aktivsubstanzen und der Zwischenprodukte

H.1 Herstellung des 4-(2,4-Difluorphenoxy)-anisols

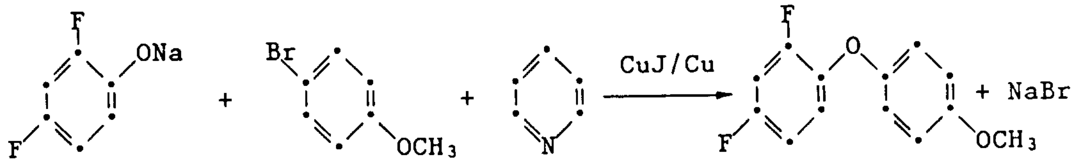


Zu einer Suspension des wasserfreien Natriumsalzes von 206 g 2,4-Difluorphenol in 400 ml Diäthylenglykoldimethyläther gibt man 5 g Kupferpulver, 5 g Kupfer(I)-jodid, 8 ml Pyridin und 393 g 4-Bromanisol. Man erhitzt das Reaktionsgemisch unter Rühren in einer Stickstoffatmosphäre während 17 Stunden auf 150-155 °C. Nach dem Abkühlen wird das Reaktionsgemisch über Hyflo filtriert, das Lösungsmittel im Vakuum weitgehend abdestilliert, der Rückstand in Aether gelöst, wiederholt mit 10 %iger Natronlauge und anschliessend mit Wasser gewaschen. Die Aetherlösung wird über Natriumsulfat getrocknet, das Lösungmittel abdestilliert und der Rückstand durch Chromatographie an Kieselgel (Eluierungsmittel: Petroläther/Diäthyläther 8:1) gereinigt. Nach Verdampfen des Lösungsmittels bleibt das Titelprodukt als Rückstand in Form eines Oels zurück, $n_D^{22}$ = 1,5466.

H.2 Herstellung des 4-(2,4-Difluorphenoxy)-phenols

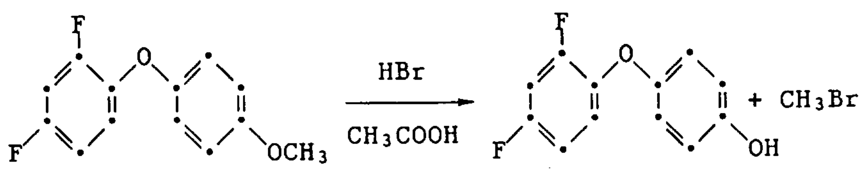


170 g des nach Beispiel H.1 erhaltenen 4-(2,4-Difluorphenoxy)-anisols werden mit 500 ml 48 %iger Bromwasserstoffsäure und 400 ml Eisessig unter Rühren während 20 Stunden auf ca. 105 °C erwärmt. Nach der Aufarbeitung wird das rohe 4-(2,4-Difluorphenoxy)-phenol durch Chromatographie an Kieselgel (Eluierungsmittel: Diäthyläther/n-Hexan/1:4) sowie durch anschliessende Umkristallisation (Diäthyläther-Petroläther) gereinigt. Die gereinigte Titelverbindung hat einen Smp. von 82-84 °C.

H.3 Herstellung der Aktivsubstanz 1-[4-(2,4-Difluorphenoxy)-phenoxy]-4-pentin (Verbindung Nr 1, Tabelle 1)

Zu einer Lösung von 8 g 4-(2,4-Difluorphenoxy)-phenol in 20 ml wasserfreiem Dimethylsulfoxid und 0,3 g fein pulverisiertes Kaliumjodid gibt man unter Kühlung die Lösung von 4,2 g Kalium-tert.-butoxid in 20 ml wasserfreiem Dimethylsulfoxid. Hierauf wird unter Rühren bei 10 °-15 °C die Lösung von 4,4 g 1-Chlor-4-pentin in 5 ml Dimethylsulfoxid zugetropft. Nach 2 Std. wird auf Raumtemperatur erwärmt (20-23 °C) und weitere 24 Std. bei dieser Temperatur gerührt. Anschliessend wird das Reaktionsgemisch auf Eiswasser gegossen und wiederholt mit Diäthyläther-Hexan (1:2) extrahiert. Die vereinigten organischen Phasen werden dreimal mit kalter 10 %iger Natronlauge und anschliessend mit Wasser neutral gewaschen, über Natriumsulfat getrocknet und das Lösungsmittel im Vakuum abdestilliert. Der Rückstand wird an Kieselgel

60 chromatographisch weiter gereinigt (Eluierungsmittel: Diäthyläther-n-Hexan 1:9), wodurch das reine 1-[4-(2,4-Difluorphenoxy)-phenoxy]-4-pentin erhalten wird. $n_D^{20}$ °: 1,5392.

In Analogie zu den obigen Beispielen wurden weitere Aktivsubstanzen noch hergestellt, wie beispielsweise:

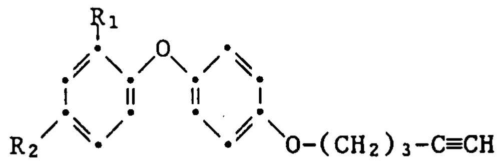

Tabelle 1

| Verbindung Nr. | $R_1$ | $R_2$ | Physik. Konst. |
|---|---|---|---|
| 1. | F | F | $n_D^{20}$ : 1,5392 |
| 2. | H | F | $n_D^{20}$ : 1,5505 |
| 3. | F | H | $n_D^{20}$ : 1,5530 |
| 4. | H | Cl | Smp.: 37-38 °C |
| 5. | Cl | H | |
| 6. | Cl | Cl | $n_D^{21}$ : 1,5785 |
| 7. | Cl | F | |
| 8. | F | Cl | |

<u>Formulierungen von Wirkstoffen der Formel I gemäss Tabelle 1</u> (% = Gewichtsprozent)

| F.1 Emulsions-Konzentrate | a) | b) |
|---|---|---|
| Wirkstoff gemäss Tabelle 1 | 10 % | 25 % |
| Ca-Dodecylbenzolsulfonat | - | 5 % |
| Ricinusöl-polyäthylenglykoläther (36 Mol AeO) | 25 % | 5 % |
| Tributylphenol-polyäthylenglykoläther (30 Mol AeO) | - | - |
| Cyclohexanon | - | - |
| Butanol | 15 % | - |
| Xylolgemisch | - | 65 % |
| Essigester | 50 % | - |

Aus solchen Konzentraten können durch Verdünnen mit Wasser Emulsionen jeder gewünschten Konzentration hergestellt werden.

| F.2 Lösungen | a) | b) |
|---|---|---|
| Wirkstoff gemäss Tabelle 1 | 10 % | 5 % |
| Polyäthylenglykol (MG 400) | 70 % | - |
| N-Methyl-2-pyrrolidon | 20 % | 20 % |
| Epoxidiertes Kokosnussöl | - | 1 % |
| Benzin (Siedegrenzen 160-190 °C) | - | 74 % |

Die Lösungen sind zur Anwendung in Form kleinster Tropfen geeignet.

| F.3 Granulate | | |
|---|---|---|
| | a) | b) |
| Wirkstoff gemäss Tabelle 1 | 5 % | 10 % |
| Kaolin | 94 % | - |
| Hochdisperse Kieselsäure | 1 % | - |
| Attapulgit | - | 90 % |

Der Wirkstoff wird in Methylenchlorid gelöst, auf den Träger aufgesprüht und das Lösungsmittel anschliessend im Vakuum abgedampft.

| F.4 Extruder Granulat | |
|---|---|
| Wirkstoff gemäss Tabelle 1 | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

| F.5 Umhüllungs-Granulat | |
|---|---|
| Wirkstoff gemäss Tabelle 1 | 3 % |
| Polyäthylenglykol (MG 200) | 3 % |
| Kaolin | 94 % |

Der feingemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

| F.6 Stäubemittel | | | | |
|---|---|---|---|---|
| | a) | b) | c) | d) |
| Wirkstoff gemäss Tabelle 1 | 2 % | 5 % | 5 % | 8 % |
| Hochdisperse Kieselsäure | 1 % | 5 % | - | - |
| Talkum | 97 % | - | 95 % | - |
| Kaolin | - | 90 % | - | 92 % |

Durch inniges Vermischen der Trägerstoffe mit dem Wirkstoff und gegebenenfalls Vermahlen auf einer geeigneten Mühle erhält man gebrauchsfertige Stäubemittel.

| F.7 Spritzpulver | | | |
|---|---|---|---|
| | a) | b) | c) |
| Wirkstoff gemäss Tabelle 1 | 20 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyäthylenglykoläther (7-8 Mol AeO) | - | 2 % | - |
| Hochdisperse Kieselsäure | 5 % | 10 % | 10 % |
| Kaolin | 67 % | 27 % | - |

Der Wirkstoff wird mit den Zusatzstoffen gut vermischt und in einer geeigneten Mühle vermahlen. Man erhält Spritzpulver, die sich mit Wasser zu Suspensionen jeder gewünschten Konzentration verdünnen

lassen.

| F.8 Suspensions-Konzentrat | |
|---|---|
| Wirkstoff gemäss Tabelle 1 | 40 % |
| Aethylenglykol | 10 % |
| Nonylphenolpolyäthylenglykoläther (15 Mol AeO) | 6 % |
| Na-Ligninsulfonat | 10 % |
| Carboxymethylcellulose | 1 % |
| 37%ige wässrige Formaldehyd-Lösung | 0,2 % |
| Silikonöl in Form einer 75%igen wässrigen Emulsion | 0,8 % |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Biologische Prüfungen

B.1 Wirkung gegen Aedes aegypti

Auf die Oberfläche von 150 ml Wasser, das sich in einem Behälter befindet, wird so viel einer 0,1%igen acetonischen Lösung des Wirkstoffes pipettiert, dass eine Konzentration von 12,5 ppm erhalten wird. Nach Verdunsten des Acetons wird der Behälter mit 30-40 2tägigen Aedes-Larven beschickt. Nach 2 und 7 Tagen wird die Mortalität geprüft.

Verbindungen gemäss Tabelle 1 zeigen gute Wirkung in diesem Test.

B.2 Wirkung auf Spodoptera littoralis und Heliothis virescens (Larven und Eier)

Es werden drei in Töpfen gezogene Baumwollpflanzen von ca. 15-20 cm Höhe mit einer sprühfähigen flüssigen Zubereitung des zu prüfenden Wirkstoffes behandelt. Nach Antrocknen des Sprühbelages werden die eingetopften Pflanzen in ein Blechgefäss von etwa 20 Litern Inhalt gestellt, das mit einer Glasplatte abgedeckt wird. Die Feuchtigkeit im Inneren des abgedeckten Gefässes wird so reguliert, dass sich kein Kondenswasser bildet. Direktes, auf die Pflanzen fallendes Licht wird vermieden. Dann werden die drei Pflanzen infestiert, und zwar insgesamt mit:

a) 50 Larven von Spodoptera littoralis oder Heliothis virescens des ersten larvalen Stadiums;
b) 20 Larven von Spodoptera littoralis oder Heliothis virescens des dritten larvalen Stadiums;
c) zwei Eispiegeln von Spodoptera littoralis oder Heliothis virescens (dazu werden je 2 Blätter einer Pflanze in einem beidseitig mit Gaze verschlossenen Plexiglaszylinder eingeschlossen); zwei Eispiegel von Spodoptera oder ein Teil eines Baumwollblattes mit darauf abgelegten Eiern von Heliothis werden zu den eingeschlossenen Blättern gegeben.

Nach 4 und 5 Tagen erfolgt die Auswertung gegenüber unbehandelten Kontrollen unter Berücksichtigung folgender Kriterien:

a) Anzahl der noch lebenden Larven,
b) larvale Entwicklungs- und Häutungshemmung,
c) Frassschaden (Schabfrass und Lochfrass),
d) Schlupfrate (Anzahl der aus den Eiern geschlüpften Larven).

Verbindungen gemäss Tabelle 1 zeigen in einer Konzentration von 400 ppm gute Gesamt-Wirksamkeit in obigem Test.

B.3 Ovizide Wirkung auf Spodoptera littoralis

Auf Filterpapier abgelegte Eier von Spodoptera littoralis werden aus dem Papier ausgeschnitten und in eine 0,05 gew.%ige Lösung des Wirkstoffes in einem Aceton/Wasser-Gemisch (1:1) getaucht. Die so behandelten Eiablagen werden dann aus diesem Gemisch herausgenommen und bei 28°C und 60 % relativer Luftfeuchtigkeit in Kunststoffschalen deponiert.

Nach 5 Tagen wird die Schlupfrate, d.h. die Anzahl Larven, die sich aus den behandelten Eiern entwickelt haben, bestimmt.

Verbindungen gemäss Tabelle 1 zeigen gute Wirkung im obigen Test.

B.4 Wirkung auf Laspeyresia pomonella (Eier)

Abgelegte Eier von Laspeyresia pomonella, die nicht älter als 24 Stunden sind, werden auf Filterpapier für 1 Minute in eine acetonisch-wässrige Lösung, enthaltend 400 ppm des zu prüfenden Wirkstoffes, eingetaucht. Nach dem Eintrocknen der Lösung wird das Filterpapier mit den Eiern in einer Petrischale ausgelegt und bei einer Temperatur von 28°C belassen. Nach 6 Tagen wird der prozentuale Schlupf aus den behandelten Eiern bewertet.

Verbindungen gemäss Tabelle 1 zeigen gute Wirkung in obigem Test.

B.5 Ovizide Wirkung auf Heliothis virescens und Spodoptera littoralis

Entsprechende Mengenanteile einer benetzbaren pulverförmigen Formulierung, enthaltend 25 Gew.% des zu prüfenden Wirkstoffes, werden mit jeweils soviel Wasser vermischt, dass sich eine wässrige Emulsion mit einer Wirkstoffkonzentration von 400 ppm ergibt.

In diese wirkstoffhaltige Emulsion werden eintägige auf Cellophan abgelegte Eigelege von Heliothis und auf Papier abgelegte Eigelege von Spodoptera während drei Minuten eingetaucht und dann auf Rundfilter abgenutscht. Die so behandelten Gelege werden in Petrischalen ausgelegt und bei 28°C und 60 % relativer Luftfeuchtigkeit in der Dunkelheit aufbewahrt. Nach 5 bis 8 Tagen wird die Schlupfrate, d.h. die Anzahl Larven, die sich aus den behandelten Eiern entwickelt haben, gegenüber unbehandelten Kontrollansätzen bestimmt.

Verbindungen gemäss Tabelle 1 zeigen in obigem Test eine 80-100%ige ovizide Wirkung (Mortalität) gegen Heliothis virescens und Spodoptera littoralis.

B.6 Vergleich der erfindungsgemässen Aktivsubstanzen mit denen des Standes der Technik hinsichtlich ovizider Wirkung auf Panonychus ulmi.

Die Verbindungen Nr. 1, 2 und 3 wurden mit folgenden Vergleichsprodukten gemäss Stand der Technik geprüft.

| Bezeichnung | Struktur | Referenz |
|---|---|---|
| A | 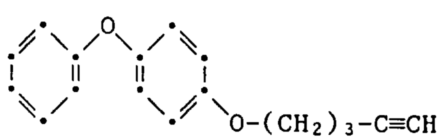 | DE-OS 2,304,962 |
| B | 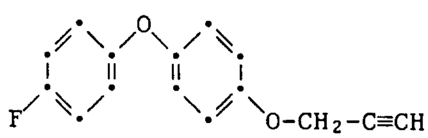 | DE-OS 2,305,698 |
| C | 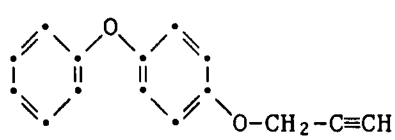 | DE-OS 2,305,698 |

Arbeitsweise zur Bestimmung der oviziden Wirkung an Panonychus ulmi.

Aus Apfelblättern werden Blattrondellen von 5 cm Durchmesser ausgestanzt. Diese Rondellen legt man auf feuchte Watte in eine Plastikpetrischale. Darauf werden pro Rondelle 7 adulte Panonychus ulmi Weibchen besiedelt und für 48 Stunden zur Eiablage darauf belassen. Nach dem Entfernen der Weibchen erfolgt die Applikation des Wirkstoffs. Mittels Handzerstäuber werden die Rondellen so lange besprüht, bis ein feiner Tröpfchenbelag entstanden ist. Pro Wirkstoff werden 2 Schalen mit den Dosierungen 50 und 25 mgAS/1 behandelt. Nach dem Trocknen der Beläge werden die Schalen zugedeckt, bei 25°C aufbewahrt und 6 Tage nach der Applikation auf % nicht geschlüpfter Eier ausgewertet (ovizide Wirkung). Der Versuch wird im Abstand von 4 Wochen 3 mal wiederholt. Vergleich der oviziden Wirkung:

Tabelle 2

| Aktivsubstanz | Dosis mg/l | % ovizid Wirkung |
|---|---|---|
| A (Stand der Technik) | 50<br>25 | 72<br>61 |
| B (Stand der Technik) | 50<br>25 | 67<br>32 |
| C (Stand der Technik) | 56<br>25 | 71<br>43 |
| 1 (gemäss Tabelle 1) | 50<br>25 | 100<br>98 |
| 2 (gemäss Tabelle 1) | 50<br>25 | 88<br>67 |
| 3 (gemäss Tabelle 1) | 50<br>25 | 93<br>78 |

B.7 Wirkung auf die Eier von Dysaphis plantaginea und von Dysaphis brancoi im Feldversuch.

Ueberwinternde Eier von Dysaphis Plantaginea ("Mehlige Apfellaus") und von Dysaphis Brancoi ("Apfelfaltenlaus") werden mit einer Spritzbrühe enthaltend 30 g Wirkstoff/100 l im Feld besprüht.

Die Auswertung erfolgt einen Monat nach dieser Applikation auf geschlüpfte und sich entwickelnde Blattläuse, bezogen auf die Zahl der total eingesetzten Eier. Dabei ergibt sich beim Einsatz der Wirkstoffe 1, 2 und 3, bei jeweils 450 g/ha Aufwandmenge folgender Anteil an geschlüpften und sich entwickelnden Tieren:
10 % Dysaphis plantaginea
4 % Dysaphis brancoi

B.8 Wirkung auf die Eier von Aphis pomi (grüne Apfelblattlaus) im Laborversuch.

Apfelzweigstücke von 10 cm Länge (2-jähriges Holz aus Winterschnitt) welche mit überwinternden Eiern von A. pomi belegt sind, werden während einer Minute in die zu prüfende Spritzbrühe, enthaltend 30 g Wirkstoff/100 Liter, eingetaucht. Die auf diese Weise behandelten Zweigstücke werden in luftdurchlässigen Versuchsgefässen bei 22°C und 60-80 % rLf während 4 Wochen aufbewahrt. Nun wird unter dem Binokular die, von den behandelten Eiern erreichte Schlupfrate festgestellt. Dabei ergibt sich für die Verbindungen 1, 2 und 3 eine ovizide Wirkung gegen Aphis pomi von >80 %.

**Patentansprüche**

**Patentansprüche für folgende Vertragsstaaten : AT, BE, CH, DE, FR, GB, GR, IT, LI, NL**

1. 1-[4-(Halophenoxy)-phenoxy]-4-pentine der Formel I

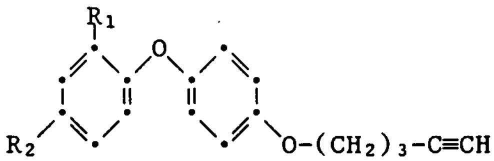

(I),

worin $R_1$ und $R_2$ voneinander unabhängig Chlor oder Fluor bedeuten und entweder $R_1$ oder $R_2$ auch Wasserstoff bedeutet.

2. Mono- und Difluorverbindungen gemäss Anspruch 1.

3. Mono- und Dichlorverbindungen gemäss Anspruch 1.

4. Das 1-[4-(4-Fluorphenoxy)-phenoxy]-4-pentin gemäss Anspruch 2.

5. Das 1-[4-(2-Fluorphenoxy)-phenoxy]-4-pentin gemäss Anspruch 2.

6. Das 1-[4-(2,4-Difluorphenoxy)-phenoxy]-4-pentin gemäss Anspruch 2.

7. Das 1-[4-(2,4-Dichlorphenoxy)-phenoxy]-4-pentin gemäss Anspruch 3.

8. Verfahren zur Herstellung einer Verbindung der Formel I, Anspruch 1, dadurch gekennzeichnet, dass man ein 4-(Halophenoxy)-phenolat der Formel II, worin $R_1$ und $R_2$ die unter Formel I angegebenen Bedeutung haben und M für ein Alkali oder Erdalkalimetall steht, mit einem 1-Halo-4-pentin der Formel III, worin $R_3$ Chlor, Brom oder Jod bedeutet,

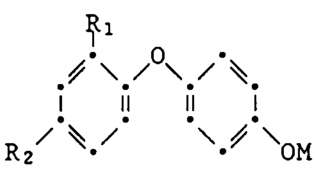

+ $R_3(CH_2)_3C{\equiv}CH$ ⟶ I + $MR_3$

II III

in einem inerten Lösungsmittel, zwischen -10 und +140 °C umsetzt und das Reaktionsprodukt isoliert.

9. Verfahren gemäss Anspruch 8, dadurch gekennzeichnet, dass es in Gegenwart eines Alkalijodids in Dimethylsulfoxid, Sulfolan oder Dimethylformamid als Lösungsmittel bei 0-80 °C durchgeführt wird und dass $R_3$ Chlor bedeutet.

10. Schädlingsbekämpfungsmittel, welches als aktive Komponente mindestens ein 1-[4-(Halophenoxy)-phenoxy]-4-pentin der Formel I

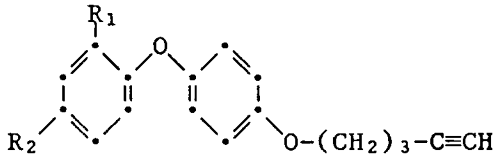

(I)

enthält, worin $R_1$ und $R_2$ voneinander unabhängig Chlor oder Fluor bedeuten und entweder $R_1$ oder $R_2$

auch Wasserstoff bedeutet.

**11.** Schädlingsbekämpfungsmittel gemäss Anspruch 10, welches als aktive Komponente 1-[4-(4-Fluorphenoxy)-phenoxy]-4-pentin enthält.

**12.** Schädlingsbekämpfungsmittel gemäss Anspruch 10, welches als aktive Komponente 1-[4-(2-Fluorphenoxy)-phenoxy]-4-pentin enthält.

**13.** Schädlingsbekämpfungsmittel gemäss Anspruch 10, welches als aktive Komponente 1-[4-(2,4-Difluorphenoxy)-phenoxy]-4-pentin enthält.

**14.** Verwendung eines 1-[4-(Halophenoxy)-phenoxy]-4-pentins der Formel I

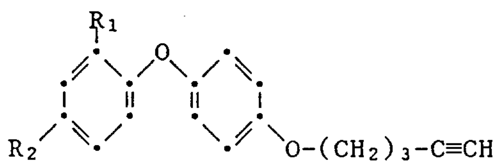

worin $R_1$ und $R_2$ voneinander unabhängig Chlor oder Fluor bedeuten und entweder $R_1$ oder $R_2$ auch Wasserstoff bedeutet zur Bekämpfung von Schädlingen an Tieren und Pflanzen.

**15.** Verwendung gemäss Anspruch 14 zur Bekämpfung von Insekten und Arachniden.

**16.** Verwendung gemäss Anspruch 15 zur Bekämpfung präimaginaler Stadien von pflanzenschädigenden Insekten.

**17.** Verwendung gemäss Anspruch 15 zur Bekämpfung der Eier von Insekten und Arachniden.

**18.** Verwendung gemäss Anspruch 17 gegen die Eier von Panonychus ulmi.

**19.** Verwendung gemäss Anspruch 14 zur Bekämpfung von Aphiden.

**20.** Verwendung gemäss Anspruch 19 zur Bekämpfung der Eier von Aphiden.

**21.** Verwendung gemäss Anspruch 19 gegen die Eier von Dysaphis plantaginea, Aphis pomi, und von Dysaphis brancoi.

**Patentansprüche für folgenden Vertragsstaat : ES**

**1.** Verfahren zur Herstellung eines 1-[4-(Halophenoxy)-phenoxy]-4-pentins der Formel I

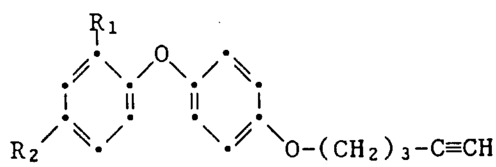

(I),

worin $R_1$ und $R_2$ voneinander unabhängig Chlor oder Fluor bedeuten und entweder $R_1$ oder $R_2$ auch Wasserstoff bedeutet, dadurch gekennzeichnet, dass man ein 4-(Halophenoxy)-phenolat der Formel II, worin $R_1$ und $R_2$ die unter Formel I angegebenen Bedeutung haben und M für ein Alkali oder Erdalkalimetall steht, mit einem 1-Halo-4-pentin der Formel III, worin $R_3$ Chlor, Brom oder Jod bedeutet,

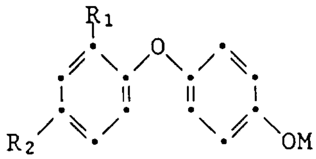

+ $R_3(CH_2)_3C\equiv CH$ → I + $MR_3$

II III

in einem inerten Lösungsmittel, zwischen -10 und +140 °C umsetzt und das Reaktionsprodukt isoliert.

**2.** Verfahren gemäss Anspruch 1, dadurch gekennzeichnet, dass man die Mono- und Difluorverbindungen der Formel I herstellt.

**3.** Verfahren gemäss Anspruch 1, dadurch gekennzeichnet, dass man die Mono- und Dichlorverbindungen der Formel I herstellt.

**4.** Verfahren gemäss Anspruch 2 zur Herstellung von 1-[4-(4-Fluorphenoxy)-phenoxy]-4-pentin.

**5.** Verfahren gemäss Anspruch 2 zur Herstellung von 1-[4-(2-Fluorphenoxy)-phenoxy]-4-pentin.

**6.** Verfahren gemäss Anspruch 2 zur Herstellung von 1-[4-(2,4-Difluorphenoxy)-phenoxy]-4-pentin.

**7.** Verfahren gemäss Anspruch 3 zur Herstellung von 1-[4-(2,4-Dichlorphenoxy)-phenoxy]-4-pentin.

**8.** Verfahren gemäss Anspruch 1, dadurch gekennzeichnet, dass es in Gegenwart eines Alkalijodids in Dimethylsulfoxid, Sulfolan oder Dimethylformamid als Lösungsmittel bei 0-80 °C durchgeführt wird und dass $R_3$ Chlor bedeutet.

**Claims**

**Claims for the following Contracting States : AT, BE, CH, DE, FR, GB, GR, IT, LI, NL**

**1.** A 1-[4-(halophenoxy)phenoxy]-4-pentyne of formula I

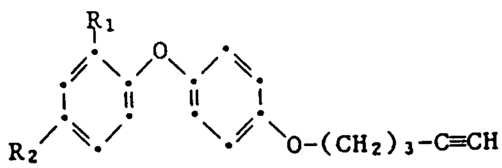

**(I)**

wherein $R_1$ and $R_2$ are each independently of the other chlorine or fluorine and one of $R_1$ and $R_2$ is also hydrogen.

**2.** A monofluoro or difluoro compound according to claim 1.

**3.** A monochloro or dichloro compound according to claim 1.

**4.** 1-[4-(4-fluorophenoxy)phenoxy]-4-pentyne according to claim 2.

**5.** 1-[4-(2-fluorophenoxy)phenoxy]-4-pentyne according to claim 2.

**6.** 1-[4-(2,4-difluorophenoxy)phenoxy]-4-pentyne according to claim 2.

**7.** 1-[4-(2,4-dichlorophenoxy)phenoxy]-4-pentyne according to claim 3.

**8.** A process for the preparation of a compound of formula I, claim 1, which comprises reacting a 4-(halophenoxy)phenolate of formula II, wherein $R_1$ and $R_2$ are as defined for formula I and M is an alkali

metal or alkaline earth metal, with a 1-halo-4-pentyne of formula III, wherein $R_3$ is chlorine, bromine or iodine:

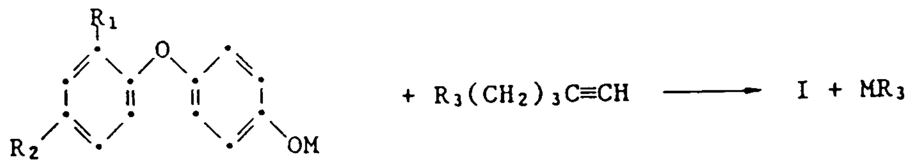


II III

in an inert solvent, at from -10 °C to +140 °C, and isolating the reaction product.

**9.** A process according to claim 8, which is carried out in the presence of an alkali metal iodide in dimethyl sulfoxide, sulfolane or dimethylformamide as solvent, at from 0 °C to 80 °C, and wherein $R_3$ is chlorine.

**10.** A pesticidal composition which comprises, as active component, at least one 1-[4-(halophenoxy)-phenoxy]-4-pentyne of formula I

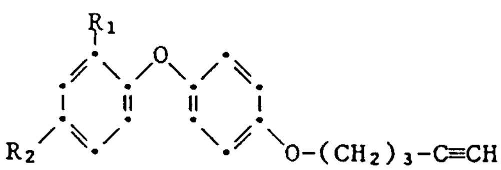

(I)

wherein $R_1$ and $R_2$ are each independently of the other chlorine or fluorine and one of $R_1$ and $R_2$ is also hydrogen.

**11.** A pesticidal composition according to claim 10, which comprises 1-[4-(4-fluorophenoxy)phenoxy]-4-pentyne as active component.

**12.** A pesticidal composition according to claim 10, which comprises 1-[4-(2-fluorophenoxy)phenoxy]-4-pentyne as active component.

**13.** A pesticidal composition according to claim 10, which comprises 1-[4-(2,4-difluorophenoxy)phenoxy]-4-pentyne as active component.

**14.** The use of a 1-[4-(halophenoxy)phenoxy]-4-pentyne of formula I

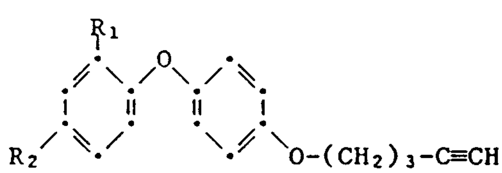

,

wherein $R_1$ and $R_2$ are each independently of the other chlorine or fluorine and one of $R_1$ and $R_2$ is also hydrogen, for controlling pests of animals and plants.

**15.** Use according to claim 14 for controlling insects and arachnids.

**16.** Use according to claim 15 for controlling pre-imaginal stages of plant-destructive insects.

**17.** Use according to claim 15 for controlling the eggs of insects and arachnids.

**18.** Use according to claim 17 against the eggs of Panonychus ulmi.

**19.** Use according to claim 14 for controlling aphids.

**20.** Use according to claim 19 for controlling the eggs of aphids.

**21.** Use according to claim 19 against the eggs of Dysaphis plantaginea, Aphis pomi and Dysaphis brancoi.

**Claims for the following Contracting State : ES**

**1.** A process for the preparation of a 1-[4-(halophenoxy)-phenoxy]-4-pentyne of formula I

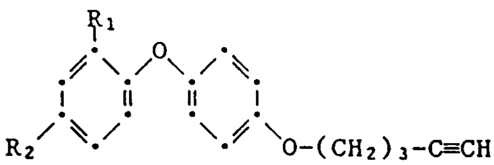

(I)

wherein $R_1$ and $R_2$ are each independently of the other chlorine or fluorine and one of $R_1$ and $R_2$ is also hydrogen, which comprises reacting a 4-(halophenoxy)-phenolate of formula II, wherein $R_1$ and $R_2$ are as defined for formula I and M is an alkali metal or alkaline earth metal, with a 1-halo-4-pentyne of formula III, wherein $R_3$ is chlorine, bromine or iodine:

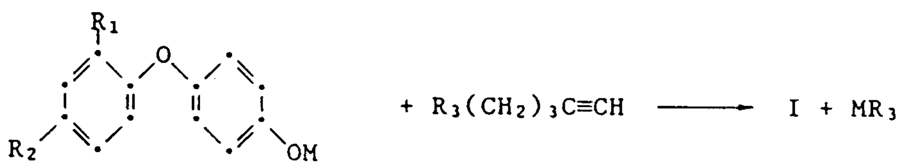


II III

in an inert solvent, at from -10 °C to +140 °C, and isolating the reaction product.

**2.** A process according to claim 1 wherein a monofluoro or difluoro compound of formula I is prepared.

**3.** A process according to claim 1 wherein a monochloro or dichloro compound of formula I is prepared.

**4.** A process according to claim 2 for the preparation of 1-[4-(4-fluorophenoxy)phenoxy]-4-pentyne.

**5.** A process according to claim 2 for the preparation of 1-[4-(2-fluorophenoxy)phenoxy]-4-pentyne.

**6.** A process according to claim 2 for the preparation of 1-[4-(2,4-difluorophenoxy)phenoxy]-4-pentyne.

**7.** A process according to claim 3 for the preparation of 1-[4-(2,4-dichlorophenoxy)phenoxy]-4-pentyne.

**8.** A process according to claim 1, which is carried out in the presence of an alkali metal iodide in dimethyl sulfoxide, sulfolane or dimethylformamide as solvent, at from 0 °C to 80 °C, and wherein $R_3$ is chlorine.

**Revendications**

**Revendications pour les Etats contractants suivants : AT, BE, CH, DE, FR, GB, GR, IT, LI, NL**

1. 1-[4-(halogénophénoxy)-phénoxy]-4-pentynes de formule I

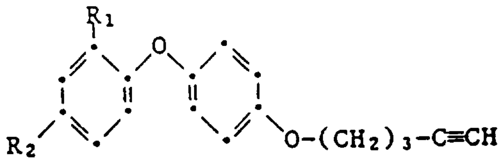

(I),

dans laquelle $R_1$ et $R_2$ représentent chacun, indépendamment l'un de l'autre, le chlore ou le fluor, l'un de ces deux symboles pouvant également représenter l'hydrogène.

2. Composés mono- et di-fluorés selon revendication 1.

3. Composés mono- et di-chlorés selon revendication 1.

4. Le 1-[4-(4-fluorophénoxy)-phénoxy]-4-pentyne selon revendication 2.

5. Le 1-[4-(2-fluorophénoxy)-phénoxy]-4-pentyne selon revendication 2.

6. Le 1-[4-(2,4-difluorophénoxy)-phénoxy]-4-pentyne selon revendication 2.

7. Le 1-[4-(2,4-dichlorophénoxy)-phénoxy]-4-pentyne selon revendication 3.

8. Procédé de préparation d'un composé de formule I de la revendication 1, caractérisé en ce que l'on fait réagir un 4-(halogénophénoxy)-phénolate de formule II, dans laquelle $R_1$ et $R_2$ ont les significations indiquées en référence à la formule I et M représente un métal alcalin ou alcalino-terreux, avec un 1-halogéno-4-pentyne de formule III dans laquelle $R_3$ représente le chlore, le brome ou l'iode,

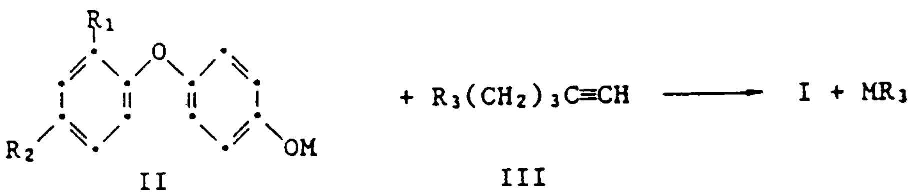

II III

dans un solvant inerte, à des températures allant de -10 à +140°C, puis on isole le produit de réaction.

9. Procédé selon revendication 8, caractérisé en ce que l'on opère en présence d'un iodure alcalin dans le diméthylsulfoxyde, le sulfolan ou le diméthylformamide servant de solvant, à des températures de 0 à 80°C, et en ce que $R_3$ représente le chlore.

10. Produit parasiticide contenant au moins un composant actif consistant en un 1-[4-(halogénophénoxy)-phénoxy]-4-pentyne de formule I

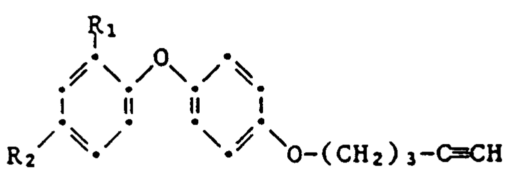

(I)

dans laquelle $R_1$ et $R_2$ représentent chacun, indépendamment l'un de l'autre, le chlore ou le fluor, l'un de ces deux symboles pouvant également représenter l'hydrogène.

**11.** Produit parasiticide selon revendication 10, contenant en tant que composant actif le 1-[4-(4-fluorophénoxy)-phénoxy]-4-pentyne.

**12.** Produit parasiticide selon revendication 10, contenant en tant que composant actif le 1-[4-(2-fluorophénoxy)-phénoxy]-4-pentyne.

**13.** Produit parasiticide selon revendication 10, contenant en tant que composant actif le 1-[4-(2,4-difluorophénoxy)-phénoxy]-4-pentyne.

**14.** Utilisation d'un 1-[4-(halogénophénoxy)-phénoxy]-4-pentyne de formule I

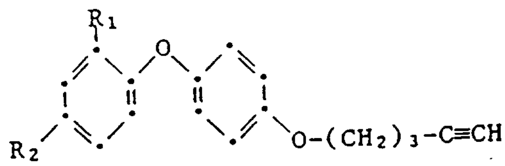

dans laquelle $R_1$ et $R_2$ représentent chacun, indépendamment l'un de l'autre, le chlore ou le fluor, l'un de ces deux symboles pouvant également représenter l'hydrogène, pour la lutte contre les parasites des animaux et des végétaux.

**15.** Utilisation selon revendication 14, pour la lutte contre les insectes et les arachnides.

**16.** Utilisation selon revendication 15, pour la lutte contre les insectes nuisibles pour les végétaux à des stades de développement précédant le stade adulte.

**17.** Utilisation selon revendication 15, pour la lutte contre les oeufs des insectes et arachnides.

**18.** Utilisation selon revendication 17, contre les oeufs de Panonychus ulmi.

**19.** Utilisation selon revendication 14, pour la lutte contre les aphidiens.

**20.** Utilisation selon revendication 19, pour la lutte contre les oeufs d'aphidiens.

**21.** Utilisation selon revendication 19, contre les oeufs de Dysaphis plantaginea, Aphis pomi et Dysaphis brancoi.

**Revendications pour l'Etat contractant suivant : ES**

**1.** Procédé de préparation d'un 1-[4-(halogénophénoxy)-phénoxy]-4-pentynede formule I

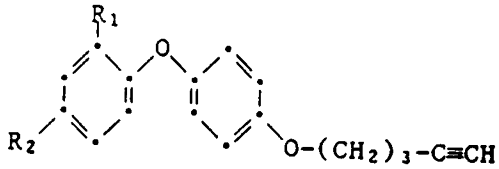

(I),

dans laquelle $R_1$ et $R_2$ représentent chacun, indépendamment l'un de l'autre, le chlore ou le fluor, l'un de ces deux symboles pouvant également représenter l'hydrogène, caractérisé en ce que l'on fait réagir un 4-(halogénophénoxy)-phénolate de formule II dans laquelle $R_1$ et $R_2$ ont les significations indiquées en référence à la formule I et M représente un métal alcalin ou alcalino-terreux, avec un 1-

halogéno-4-pentyne de formule III dans laquelle $R_3$ représente le chlore, le brome ou l'iode

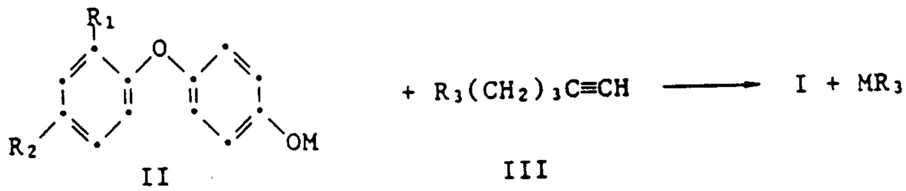


II III

dans un solvant inerte, à des températures allant de -10 à +140°C, puis on isole le produit de réaction.

**2.** Procédé selon revendication 1, caractérisé en ce que l'on prépare les composés mono- et di-fluorés de formule I.

**3.** Procédé selon revendication 1, caractérisé en ce que l'on prépare les composés mono- et di-chlorés de formule I.

**4.** Procédé selon revendication 2, pour la préparation du 1-[4-(4-fluorophénoxy)-phénoxy]-4-pentyne.

**5.** Procédé selon revendication 2, pour la préparation du 1-[4-(2-fluorophénoxy)-phénoxy]-4-pentyne.

**6.** Procédé selon revendication 2, pour la préparation du 1-[4-(2,4-difluorophénoxy)-phénoxy]-4-pentyne.

**7.** Procédé selon revendication 3, pour la préparation du 1-[4-(2,4-dichlorophénoxy)-phénoxy]-4-pentyne.

**8.** Procédé selon revendication 1, caractérisé en ce que l'on opère en présence d'un iodure alcalin dans le sulfoxyde de diméthyle, le sulfolan ou le diméthylformamide servant de solvant, à des températures de 0 à 80°C, et en ce que $R_3$ représente le chlore.